(19) Europäisches Patentamt
European Patent Office
Office européen des brevets

(11) EP 0 601 302 B1

(12) **EUROPÄISCHE PATENTSCHRIFT**

(45) Veröffentlichungstag und Bekanntmachung des Hinweises auf die Patenterteilung:
**10.07.1996 Patentblatt 1996/28**

(51) Int. Cl.[6]: **A61K 7/13**, C09B 29/036, C09B 29/09, C09B 67/00

(21) Anmeldenummer: **93116213.5**

(22) Anmeldetag: **07.10.1993**

(54) **Mittel zum Färben von Haaren mit einem Gehalt an 3-Pyridinazofarbstoffen sowie neue 3-Pyridinazofarbstoffe**

Hair dyeing composition containing 3-pyridinazo dyes and novel 3-pyridazo dyes

Composition pour la teinture des cheveux contenant des colorants de type pyridinazoiques et nouveaux colorants de type pyridinazoiques

(84) Benannte Vertragsstaaten:
**DE ES FR GB IT**

(30) Priorität: **07.12.1992 DE 4241173**

(43) Veröffentlichungstag der Anmeldung:
**15.06.1994 Patentblatt 1994/24**

(73) Patentinhaber: **Wella Aktiengesellschaft**
**D-64295 Darmstadt (DE)**

(72) Erfinder:
- **Löwe, Isolde**
  **D-64625 Bensheim (DE)**
- **Clausen, Thomas, Dr.**
  **D-64665 Alsbach (DE)**
- **Balzer, Wolfgang R., Dr.**
  **D-64665 Alsbach (DE)**

(56) Entgegenhaltungen:
**FR-A- 1 585 308**
**FR-A- 2 285 851**
**GB-A- 1 153 196**
**GB-A- 2 165 257**
**US-A- 3 082 200**

EP 0 601 302 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Gegenstand der Erfindung sind ein Mittel zum Färben von Haaren mit einem Gehalt an 3-Pyridinazofarbstoffen sowie neue 3-Pyridinazofarbstoffe.

Für das Färben von Haaren gewinnen neben den Oxidationshaarfarbstoffen, die durch oxidative Kupplung einer oder mehrerer Entwicklerkomponenten untereinander gebildet werden, in zunehmendem Maße direktziehende Haarfarbstoffe an Bedeutung. Direktziehende Farbstoffe bieten den Vorteil, daß sie ohne Zusatz von Oxidationsmitteln (beispielsweise Wasserstoffperoxid) zur Anwendung kommen und deshalb das Haar während des Färbevorganges wesentlich weniger schädigen.

Gute Haarfärbemittel müssen eine Vielzahl von Anforderungen erfüllen. So müssen sie die gewünschten Farbnuancen in ausreichender Intensität ausbilden und gleichmäßig auf das Haar aufziehen, ohne die Kopfhaut zu stark anzufärben. Die erzeugten Haarfärbungen müssen weiterhin eine gute Reibeechtheit und eine ausreichende Stabilität gegen Licht, Wärme, Schweiß, Haarreinigungsmittel und die bei der Dauerverformung der Haare verwendeten Chemikalien aufweisen sowie gegen die Einwirkung von sauren Mitteln und verdünnten Säuren stabil sein. Schließlich sollen diese Mittel in toxikologischer und dermatologischer Hinsicht unbedenklich sein.

Weiterhin müssen die verwendeten Farbstoffe bestimmte Anforderungen bezüglich ihres Farbcharakters erfüllen, damit die unterschiedlichen Farbnuancen erzielt werden können. So werden beispielsweise für modische Farbnuancen Farbstoffe bevorzugt, die klare, leuchtende Farben ergeben, während für die Nuancierung von Naturtönen Farbstoffe verwendet werden, die eine derartige Farbbrillanz nicht besitzen.

Der für die Nuancierung von Haarfarben am häufigsten verwendete gelbe Azofarbstoff ist das 4-Amino-4'-[bis-(β-hydroxyethyl)-amino]-azobenzol (DISPERSE BLACK 9).

Obwohl das 4-Amino-4'-[bis-(β-hydroxyethyl)-amino]-azobenzol häufig in Haarfärbemitteln eingesetzt wird, ist dieser Farbstoff in anwendungstechnischer Hinsicht nicht völlig zufriedenstellend. Zum einen besteht der Verdacht, daß diese Verbindung mutagen ist, und zum anderen besitzen die mit dieser Verbindung erzielten Haarfärbungen, ebenso wie die mit dem aus der DE-OS 25 43 100 bekannten 3-[4'-N,N-Bis-(β-hydroxyethyl)amino-phenylazo]-pyridin erzielten Haarfärbungen, nur eine geringe Stabilität gegenüber der Einwirkung von Säuren und sauren Zubereitungen, wie zum Beispiel sauren Haarspülungen und Shampoos, sauren Dauerwellmitteln und Fixierungen.

Es bestand daher die Aufgabe, Haarfärbemittel mit einem Gehalt an einem gelben Azofarbstoff zur Verfügung zu stellen, die die vorstehend aufgeführten Anforderungen an Haarfarbstoffe erfüllen und bei denen insbesondere gegen Säuren und saure Zubereitungen, wie zum Beispiel saure Haarspülungen oder Haarreinigungsmittel, saure Dauerwellmittel und Fixiermittel, stabile und dadurch dauerhaftere und über einen längeren Zeitraum gleichbleibende Färbungen der Haare erzielt werden.

Es wurde nunmehr gefunden, daß durch ein Mittel zum Färben von Haaren mit einem Gehalt an bestimmten 3-Pyridinazofarbstoffen der Formel (I) oder (II) die gestellte Aufgabe in hervorragender Weise gelöst wird.

Gegenstand der vorliegenden Erfindung ist daher ein Mittel zum Färben von Haaren mit einem Gehalt an für Haarfärbemittel üblichen Zusätzen, welches dadurch gekennzeichnet ist, daß es mindestens einen 3-Pyridinazofarbstoff der Formel (I) oder (II)

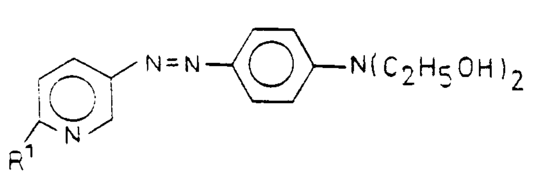

(I)

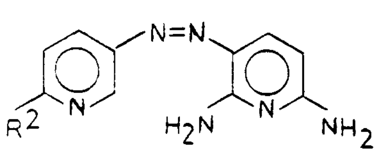

(II)

wobei $R^1$ eine Aminogruppe, eine $C_1$- bis $C_6$-Alkylaminogruppe oder eine $C_2$- bis $C_4$-Hydroxyalkylaminogruppe bedeutet und $R^2$ Wasserstoff, eine Aminogruppe, eine $C_1$- bis $C_6$-Alkylaminogruppe oder eine $C_2$- bis $C_4$-Hydroxyalkylaminogruppe ist, enthält.

Von den erfindungsgemäßen Mitteln ist jenes bevorzugt, welches mindestens einen der folgenden 3-Pyridinazofarbstoffe der Formel (I) oder (II) enthält: 2,6-Diamino-3-[3'-azopyridyl]-pyridin, 2-Amino-5-[4'-N,N-bis-(β-hydroxyethyl)amino-phenylazo]-pyridin, 2-Methylamino-5-[4'-N,N-bis-(β-hydroxyethyl)amino-phenylazo]-pyridin und 2-(β-Hydroxyethyl)amino-5-[4'-N,N-bis-(β-hydroxyethyl)amino-phenylazo]-pyridin.

Die Farbstoffe der Formel (I) oder (II) sollen in dem erfindungsgemäßen Mittel in einer Konzentration von 0,01 bis 1 Gewichtsprozent, vorzugsweise in einer Konzentration von 0,01 bis 0,4 Gewichtsprozent, enthalten sein.

Bei dem erfindungsgemäßen Haarfärbemittel handelt es sich um ein Mittel, das mindestens einen Farbstoff der Formel (I) oder (II) enthält oder aber um ein Mittel, welches zusätzlich zu mindestens einem Farbstoff der Formel (I) oder (II) noch einen oder mehrere weitere direkt auf das Haar aufziehende Farbstoffe enthält. Von diesen direkt auf das Haar aufziehenden Farbstoffen seien beispielsweise die folgenden erwähnt:
aromatische Nitrofarbstoffe, wie zum Beispiel 2-Nitro-1,4-diaminobenzol, 2-Amino-4,6-dinitrophenol, 2-Amino-5-(β-hydroxyethyl)amino-nitrobenzol, 4-(β-Hydroxyethyl)-amino-3-nitrophenol, 1-(β-Hydroxyethyl)amino-2-amino-4-nitrobenzol, 4-[(2',3'-Dihydroxypropyl)amino]-3-nitrotrifluormethylbenzol, 1,4-Bis-[(β-hydroxyethyl)amino]-4-N-ethyl-2-nitrobenzol, 4-(β-Hydroxyethyl)amino-3-nitrotoluol, 2,5-Bis-[(β-hydroxyethyl)amino]-nitrobenzol, 2-(β-Hydroxyethyl)amino-4,6-dinitrophenol, 4-[(2',3'-Dihydroxypropyl)amino]-1-[(β-hydroxyethyl)amino]-2-nitrobenzol, 2-Amino-6-chlor-4-nitrophenol, 4-[(β-Hydroxyethyl)amino]-2-nitro-anilin, 1-[(β-Hydroxyethyl)amino]-4-[bis-(β-hydroxyethyl)amino]-2-nitro-benzol, 4-[Bis-(β-hydroxyethyl)amino]-1-[(3'-hydroxypropyl)amino]-2-nitrobenzol, 1-[(2',3'-Dihydroxypropyl)amino]-4-[N-ethyl-N-(β-hydroxyethyl)amino]-2-nitro-benzol, 4-(β-Ureidoethyl)-amino-nitrobenzol, 1-Amino-4-[(2',3'-dihydroxypropyl)amino]-5-chlor-2-nitro-benzol und 1-Amino-2-nitro-4-[bis-(β-hydroxyethyl)amino]-benzol; Triphenylmethanfarbstoffe, wie zum Beispiel Basic Violet 1 (C.I. 42 535), Basic Violet 14 (C.I. 42 510) und Basic Violet 2 (C.I. 42 520); Azofarbstoffe, wie beispielsweise Acid Brown 4 (C.I. 14 805); Anthrachinonfarbstoffe, wie zum Beispiel Disperse Violet 4 (C.I. 61 105), 1,4,5,8-Tetra-aminoanthrachinon (C.I. 64 500), 1-Methylamino-4-[(β-hydroxyethyl)amino]-anthrachinon, 1,4-Diaminoanthrachinon und 1,4-[Bis-(2',3'-dihydroxypropyl)amino]-anthrachinon, wobei die Farbstoffe dieser Klassen je nach Art ihrer Substituenten sauren, nichtionogenen oder basischen Charakter haben können. Weitere geeignete, direkt auf das Haar aufziehende Farbstoffe sind beispielsweise in dem Buch von J.C. Johnson, "Hair Dyes", Noyes Data Corp., Park-Ridge (USA) (1973), Seiten 3 bis 91 und 113 bis 139 (ISBN: 0-8155-0477-2) beschrieben.

Der Gesamtgehalt an Haarfarbstoffen soll in dem erfindungsgemäßen Mittel vorzugsweise 0,01 bis 3 Gewichtsprozent betragen.

Die Zubereitungsform des hier beschriebenen Haarfärbemittels, welches häufig auch als Tönungsmittel bezeichnet wird, kann beispielsweise die einer Lösung, insbesondere einer wäßrigen oder wäßrig-alkoholischen Lösung, sein. Bevorzugte Zubereitungsformen sind weiterhin die einer Creme, eines Geles, einer Emulsion oder die eines Schaumes, wobei sie auch im Gemisch mit einem Treibgas oder mittels einer Pumpe versprüht werden können.

Der pH-Wert dieses Färbemittels liegt im Bereich von 3 bis 10,5, insbesondere bei pH 5,5 bis 9,5, wobei die Einstellung des gewünschten pH-Wertes entweder mit Ammoniak oder organischen Aminen, wie beispielsweise Monoethanolamin oder Diethanolamin, oder aber Säuren, wie zum Beispiel Salzsäure, Zitronensäure oder Weinsäure, erfolgt.

Die Verwendung des hier beschriebenen Haarfärbemittels erfolgt üblicherweise durch Aufbringen einer für die Haarfärbung ausreichenden Menge des Mittels auf die Haare, mit denen es 5 bis 30 Minuten lang in Berührung bleibt.

Anschließend wird mit Wasser, gegebenenfalls noch mit einer wäßrigen Lösung einer schwachen organischen Säure, gespült und sodann getrocknet.

Als schwache organische Säure können beispielsweise Essigsäure, Zitronensäure, Weinsäure und ähnliche Verwendung finden.

Das erfindungsgemäße Haarfärbemittel kann in Form eines Haarfärbemittels mit zusätzlicher Haarfestigung vorliegen, das mindestens ein in der Kosmetik üblicherweise verwendetes Polymerisat oder natürliches Polymer enthält. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungen wie Polyacrylsäure beziehungsweise Polymethacrylsäure, basische Polymerisate der Ester der Polyacrylsäure oder Polymetharylsäure mit Aminoalkoholen bezie-hungsweise deren Salze oder Quaternisierungsprodukte, Polyacrylnitril, Polyvinyllactame sowie Copolymerisate aus diesen Verbindungen wie zum Beispiel Polyvinylpyrrolidon-Vinylacetat und dergleichen erwähnt.

Auch natürliche Polymere, wie zum Beispiel Chitosan (entacetyliertes Chitin) oder Chitosanderivate, können für den genannten Zweck eingesetzt werden.

Die Polymerisate und natürlichen Polymere sind in dem zuvor beschriebenen Haarfärbemittel in der für solche Mittel üblichen Menge von 1 bis 5 Gewichtsprozent enthalten. Der pH-Wert des Mittels liegt im Bereich von etwa 6,0 bis 9,0. Die Anwendung dieses Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel gegebenenfalls weitere, für Haarfärbemittel übliche Zusätze, wie zum Beispiel Konservierungsstoffe und Parfümöle, Lösungsmittel wie Wasser, niedere aliphatische Alkohole, beispielsweise Ethanol, Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Nonylphenole, Fettsäurealkanolamide, oxethylierte Fettalkohole, oxethylierte Fettsäureester, ferner Verdicker wie höhere Fettalkohole, Stärke oder Cellulosederivate, weiterhin Weichmacher, Vaseline, Paraffinöl und Fettsäuren sowie außerdem Pflegestoffe wie kationische Harze, Lanolin-derivate, Cholesterin, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,5 bis 30 Gewichtsprozent, die Verdicker in einer Menge von 0,1 bis 25 Gewichtsprozent und die Pflegestoffe in einer Konzentration von 0,1 bis 5,0 Gewichtsprozent.

Die in den hier beschriebenen Haarfärbemitteln enthaltenen Verbindungen der Formel (I) oder (II) besitzen ein gutes Aufziehvermögen beziehungsweise Ausgleichsvermögen und eine hervorragende Stabilität gegenüber Säuren und sauren Zubereitungen.

Hinsichtlich der färberischen Möglichkeiten ermöglichen diese Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt.

So ergeben beispielsweise die Verbindungen der Formel (II) rein gelbe Ausfärbungen mit hoher Brillianz, während die Verbindungen der Formel (I) gelbe, leicht braunstichige Ausfärbungen ergeben.

Gegenstand der Erfindung sind weiterhin die neuen 3-Pyridinazofarbstoffe der Formel (I), wobei insbesondere 2-Amino-5-[4'-N,N-bis-(β-hydroxyethyl)amino-phenylazo]-pyridin, 2-Methylamino-5-[4'-N,N-bis-(β-hydroxyethyl)amino-phenylazo]-pyridin und 2-(β-Hydroxyethyl)-amino-5-[4'-N,N-bis-(β-hydroxyethyl)amino-phenylazo]-pyridin zu nennen sind.

Die neuen 3-Pyridinazofarbstoffe der Formel (I) lassen sich ebenso wie die 3-Pyridinazofarbstoffe der Formel (II) mit Hilfe von bekannten Verfahren durch Diazotierung von 3-Amino-pyridinderivaten und anschließende Kupplung des erhaltenen Diazoniumsalzes mit einem geeigneten N,N-Bis-(β-hydroxyethyl)anilin beziehungsweise 2,6-Diaminopyridin herstellen.

So können die Verbindungen der Formel (I) beispielsweise durch analoge Anwendung der aus der DE-OS 25 43 100 und DE-PS 11 11 316 bekannten Verfahren hergestellt werden, während die Herstellung der Verbindungen der Formel (II) zum Beispiel in Analogie zu der aus der DE-PS 543 288 bekannten Vorschrift erfolgen kann.

Die nachfolgenden Beispiele sollen den Gegenstand der vorliegenden Erfindung näher erläutern, ohne diesen hierauf zu beschränken.

## Herstellungsbeispiele

### Beispiel 1: Herstellung von 2-Amino-5-[(4'-N,N-bis-(β-hydroxyethyl)amino-phenylazo]-pyridin

49 g (0,33 mol) 2-Acetamino-5-amino-pyridin werden in 162 ml 50%iger Salzsäure gelöst und mit 22,3 g (0,33 mol) Natriumnitrit unter Kühlung diazotiert. Zu der erhaltenen Lösung des Diazoniumsalzes wird sodann tropfenweise eine Lösung von 58 g (0,32 mol) N,N-Bis-(β-hydroxyethyl)amino-benzol in 58 ml Essigsäure gegeben. Nach Beendigung der Reaktion wird die Reaktionsmischung mit 98 g Natriumacetat versetzt, der erhaltene Niederschlag abfiltriert und mit Wasser gewaschen.

Anschließend wird der Niederschlag in 600 ml 10 %iger wäßriger Natronlauge 2 Stunden lang auf 80 °C erwärmt. Nach Abkühlung der Reaktionsmischung wird der Niederschlag abfiltriert und mit Wasser gewaschen.

Nach Umkristallisation aus Ethanol werden 80 g (= 81 % der theoretischen Ausbeute) gelbliche Kristalle mit einem Schmelzpunkt von 175 °C erhalten.

$^1$H-NMR (300 MHz; DMSO-$d_6$):
δ = 8,42 ppm (d, J = 2,5 Hz, 1 H, 6-H),
7,80 ppm (dd, J = 2,5 Hz und 8,9 Hz, 1 H, 4-H),
7,64 ppm (d, J = 9 Hz, 2 H, 2'-H),
6,89 ppm (d, J = 9 Hz, 2 H, 3'-H),
6,61 ppm (s, 2 H, $NH_2$, tauscht mit $D_2O$ aus),
6,53 ppm (d, J = 8,9 Hz, 1 H, 3-H),
3,51 - 3,59 ppm (m, 8 H, $\underline{CH_2\text{-}CH_2}$) und
4,83 ppm (t, J = 5,2 Hz, 2 H, OH, tauscht mit $D_2O$ aus)
MS (70 eV): m/e = 301 ($M^+$)

### Beispiel 2: Herstellung von 2-Methylamino-5-[4'-N,N-bis-(β-hydroxyethyl)amino-phenylazo]-pyridin

#### Stufe 1: Synthese von 2-(N-Ethyloxycarbonyl-N-methyl)amino-5-nitro-pyridin

40 g (0,25 mol) 2-Methylamino-5-nitro-pyridin werden in 300 ml Dioxan mit 27 g (0,25 mol) Chlorameisensäureethylester und 9,6 g (0,13 mol) Calciumhydroxid umgesetzt. Nach beendeter Reaktion wird die Reaktionsmischung filtriert und das Filtrat eingeengt. Sodann wird der Rückstand in Wasser gegossen und der ausgefallene Niederschlag abgesaugt und getrocknet.

Es werden 48 g (= 82 % der theoretischen Ausbeute) leicht gelblich gefärbte Kristalle mit einem Schmelzpunkt von 94 °C erhalten.

**Stufe 2: Synthese von 2-(N-Ethyloxycarbonyl-N-methyl)amino-5-amino-pyridin**

23,5 g (0,1 mol) 2-(N-Ethyloxycarbonyl-N-methyl)amino-5-nitro-pyridin werden in 300 ml Methanol gelöst und mit 0,1 g eines Palladium/Aktivkohle-Katalysators (10 %) bei Raumtemperatur (20 bis 30 °C) und Normaldruck hydriert, bis die berechnete Menge an Wasserstoff aufgenommen worden ist. Nach Beendigung der Reaktion wird der Katalysator abfiltriert und das Lösungsmittel im Vakuum abdestilliert.

Man erhält 21,5 g (= 95 % der theoretischen Ausbeute) eines farblosen Öls.

**Stufe 3: Synthese von 2-Methylamino-5-[4'-N,N-bis-(β-hydroxyethyl)amino-phenylazo]-pyridin**

9,5 g (0,05 mol) 2-(N-Ethyloxycarbonyl-N-methyl)amino-5-amino-pyridin werden in 25 ml 50 %iger Salzsäure gelöst und mit 3,45 g (0,05 mol) Natriumnitrit unter Kühlung diazotiert. Zu der erhaltenen Lösung des Diazoniumsalzes wird sodann tropfenweise eine Lösung von 9 g (0,05 mol) N,N-Bis-(β-hydroxyethyl)amino-benzol in 9 ml Essigsäure gegeben. Nach Beendigung der Reaktion wird die Reaktionsmischung mit 30 g Natriumacetat versetzt, der erhaltene Niederschlag abfiltriert und mit Wasser gewaschen.

Anschließend wird der Niederschlag in 200 ml 20 %iger wäßriger Natronlauge 4 Stunden lang unter Rückfluß erwärmt. Nach Abkühlung der Reaktionsmischung wird der Niederschlag abfiltriert und mit Wasser gewaschen.

Es werden 5 g (= 32 % der theoretischen Ausbeute) gelbliche Kristalle mit einem Schmelzpunkt von 194 bis 196 °C erhalten.

$^1$H-NMR (300 MHz, DMSO-$d_6$):
δ = 8,50 ppm (d, J = 2,3 Hz, 1 H, 6-H),
7,81 ppm (dd, J = 2,3 Hz und 9,1 Hz, 1 H, 4-H),
7,64 ppm (d, J = 9 Hz, 2 H, 2'-H),
7,21 ppm (q, J = 4,8 Hz, 1 H, NH, tauscht mit $D_2O$ aus)
6,78 ppm (d, J = 9 Hz, 2 H, 3'-H),
6,54 ppm (d, J = 9,1 Hz, 1 H, 3-H),
4,85 ppm (s, 2 H, <u>OH</u>,tauscht mit $D_2O$ aus) aus)
3,41 - 3,58 ppm (m, 8 H, $\underline{CH_2}$-$\underline{CH_2}$) und
2,86 ppm (d, J = 4,8 Hz, 3 H, $\underline{CH_3}$, nach $D_2O$-Austausch s)
MS (70 eV): m/e = 315 ($M^+$)

**Beispiel 3: 2-(β-Hydroxyethyl)amino-5-[4'-N,N-bis-(β-hydroxyethyl)amino-phenylazo]-pyridin**

**Stufe 1: Synthese von 2-[N-Ethyloxycarbonyl-N-(2'-ethyloxycarbonyloxy-ethyl)amino]-5-nitro-pyridin**

10 g (0,055 mol) 2-(β-Hydroxyethyl)amino-5-nitro-pyridin werden in 150 ml Dioxan mit 12 g (0,11 mol) Chlorameisensäureethylester und 4 g (0,055 mol) Calciumhydroxid auf 70 °C erwärmt. Anschließend wird die Reaktionsmischung filtriert und das Filtrat eingedampft.

Es werden 13 g (= 72 % der theoretischen Ausbeute) schwach gelbe Kristalle mit einem Schmelzpunkt von 54 °C erhalten.

**Stufe 2: Synthese von 2-[N-Ethyloxycarbonyl-N-(2'-ethyloxycarbonyloxy-ethyl)amino]-5-amino-pyridin**

9,8 g (0,03 mol) 2-[N-Ethyloxycarbonyl-N-(2'-ethyloxy-carbonyloxy-ethyl)amino]-5-amino-pyridin werden in 100 ml Methanol gelöst und mit 0,1 g eines Palladium/Aktivkohle-Katalysators (10 %) bei Raumtemperatur (20 bis 30 °C) und Normaldruck hydriert, bis die berechnete Menge an Wasserstoff aufgenommen worden ist. Nach Beendigung der Reaktion wird der Katalysator abfiltriert und das Lösungsmittel im Vakuum abdestilliert.

Man erhält 8,6 g (= 97 % der theoretischen Ausbeute) eines farblosen Öls.

**Stufe 3: Synthese aus 2-(β-Hydroxyethyl)amino-5-[4'-N,N-bis-(β-hydroxyethyl)amino-phenylazo]-pyridin**

5,9 g (0,02 mol) 2-[N-Ethyloxycarbonyl-N-(2'-ethyloxy-carbonyloxy-ethyl)-amino]-5-amino-pyridin werden in 10 ml 50 %iger Salzsäure gelöst und mit einer Lösung von 1,25 g (0,02 mol) Natriumnitrit in 15 ml Wasser unter Kühlung diazotiert. Zu der erhaltenen Lösung des Diazoniumsalzes wird sodann tropfenweise eine Lösung von 3,62 g (0,02 mol) N,N-Bis-(β-hydroxyethyl)amino-benzol in 3 ml Essigsäure gegeben. Nach Beendigung der Reaktion wird die Reaktionsmischung mit 10 g Natriumacetat versetzt, der erhaltene Niederschlag abfiltriert und mit Wasser gewaschen.

Anschließend wird der Niederschlag in 100 ml 10 %iger wäßriger Natronlauge 2 Stunden lang unter Rückfluß erwärmt. Nach Abkühlung der Lösung wird der Niederschlag abfiltriert.

Es werden 4 g (= 58 % der theoretischen Ausbeute) schwach gelb gefärbte Kristalle mit einem Schmelzpunkt von 148 °C erhalten.
$^1$H-NMR (300 MHz, DMSO-$d_6$):
δ = 8,47 ppm (s, 1 H, 6-H),
7,80 ppm (d, J = 9,1 Hz, 1 H, 4-H),
7,64 ppm (d, J = 8,4 Hz, 2 H, 2'-H),
7,25 ppm (s, 1 H, NH, tauscht mit $D_2O$ aus),
6,79 ppm (d, J = 8,5 Hz, 2 H, 3'-H),
6,60 ppm (d, J = 9,1 Hz, 1 H, 3-H),
4,85 ppm (s, 3 H, OH, tauscht mit $D_2O$ aus) und
3,41 - 3,58 ppm (m, 12 H, $CH_2$-$CH_2$)
MS (70 eV): m/e = 345 ($M^+$)

Für sämtliche $^1$H-NMR-Spektren gilt:
s = Singulett; d = Dublett; t = Triplett;
q = Quartett; m = Multiplett

**Haarfärbebeispiele**

**Beispiel 4: Haarfärbemittel in Cremeform**

| | |
|---|---|
| 1,100 g | 1,4-Bis-[(β-hydroxyethyl)amino]-4-N-ethyl-2-nitro-benzol-Hydrochlorid |
| 0,170 g | 4-(β-Hydroxyethyl)amino-3-nitro-toluol |
| 0,100 g | 2-Amino-5-[4'-N,N-bis-(β-hydroxyethyl)-amino-phenylazo]-pyridin |
| 0,047 g | 1,4-Bis-[(2',3'-dihydroxypropyl)amino]-anthrachinon |
| 0,040 g | 1,4-Diaminoanthrachinon |
| 7,500 g | Cetylalkohol |
| 1,750 g | Laurylalkoholdiglykolethersulfat-Natriumsalz (28 %ige wäßrige Lösung) |
| 0,200 g | Ammoniak (25 %ige wäßrige Lösung) |
| 0,100 g | p-Hydroxybenzoesäuremethylester |
| 88,993 g | Wasser |
| 100,000 g | |

50 g des vorstehenden Haarfärbemittels werden auf weiße Naturhaare aufgetragen. Nach einer Einwirkungszeit von 20 Minuten bei Raumtemperatur wird das Haar mit Wasser gespült und getrocknet. Das Haar ist in einem intensiven schwarzen Farbton gefärbt.

**Beispiel 5: Flüssiges Haarfärbemittel**

| | |
|---|---|
| 0,30 g | 2-(β-Hydroxyethyl)amino-5-[bis-(β-hydroxyethyl)amino]-nitrobenzol |
| 0,08 g | 2-(β-Hydroxyethyl)amino-5-[4'-N,N-bis-(β-hydroxyethyl)amino-phenylazo]-pyridin |
| 0,05 g | 4-(β-Ureidoethyl)amino-nitrobenzol |
| 0,04 g | 1,4-Diaminoanthrachinon |
| 0,04 g | 1-Methylamino-4-(β-hydroxyethyl)amino-anthrachinon |
| 10,00 g | Isopropanol |
| 10,00 g | Ammoniak (25 %ige wäßrige Lösung) |
| 5,00 g | Laurylalkoholdiglykolethersulfat-Natriumsalz (28 %ige wäßrige Lösung) |
| 0,50 g | Hydroxyethylcellulose |
| 73,99 g | Wasser |
| 100,00 g | |

Gebleichtes Naturhaar wird 20 Minuten lang bei Raumtemperatur mit dem vorstehenden Haarfärbemittel behandelt. Anschließend wird das Haar mit Wasser gespült und getrocknet. Das Haar hat eine dunkelgraue Färbung erhalten.

**Beispiel 6: Flüssiges Haarfärbemittel**

| | |
|---|---|
| 0,07 g | 1,4-Bis-[(β-hydroxyethyl)amino]-4-N-ethyl-2-nitro-benzol-Hydrochlorid |
| 0,06 g | 4-(β-Hydroxyethyl)amino-3-nitro-toluol |
| 0,04 g | 1-Methylamino-4-(β-hydroxyethyl)amino-anthrachinon |
| 0,04 g | 1-Amino-4-[(2',3'-dihydroxypropyl)amino]-5-chlor-2-nitro-benzol |
| 0,03 g | 2-Methylamino-5-[4'-N,N-bis-(β-hydroxyethyl)amino-phenylazo]-pyridin |
| 10,00 g | Isopropanol |
| 10,00 g | Ammoniak (25 %ige wäßrige Lösung) |
| 5,00 g | Laurylalkoholdiglykolethersulfat-Natriumsalz (28 %ige wäßrige Lösung) |
| 0,50 g | Hydroxyethylcellulose |
| 74,26 g | Wasser |
| 100,00 g | |

Gebleichtes Naturhaar wird 20 Minuten lang bei Raumtemperatur mit dem vorstehenden Haarfärbemittel behandelt. Anschließend werden die Haare mit Wasser gespült und getrocknet.

Das so behandelte Haar besitzt eine dunkelblonde Färbung.

**Beispiel 7: Haarfärbemittel in Cremeform**

| | |
|---|---|
| 0,14 g | 4-(β-Hydroxyethyl)amino-3-nitro-toluol |
| 0,10 g | 1-(β-Hydroxyethyl)amino-4-[bis-(β-hydroxyethyl)amino]-2-nitro-benzol |
| 0,05 g | 1-Amino-4-[(2',3'-dihydroxypropyl)amino]-5-chlor-4-nitro-benzol |
| 0,03 g | 2,6-Diamino-3-(3'-azopyridyl)-pyridin |
| 7,00 g | Cetylalkohol |
| 1,20 g | Laurylalkoholdiglykolethersulfat-Natrium salz (28 %ige wäßrige Lösung) |
| 0,10 g | Ammoniak (25 %ige wäßrige Lösung) |
| 91,38 g | Wasser |
| 100,00 g | |

50 g der vorstehenden Haarfärbecreme werden auf weißes Naturhaar aufgetragen und nach einer Einwirkungszeit von 20 Minuten mit Wasser ausgespült. Anschließend werden die Haare getrocknet.

Das so behandelte Haar ist in einem intensiven schwarzen Farbton gefärbt.

**Beispiel 8: Vergleichsversuche zur Säurebeständigkeit**

Gebleichtes Naturhaar wurde 20 Minuten lang bei 40 °C mit dem nachfolgenden Haarfärbemittel, in dem jeweils der Farbstoff (A) bis (F) variiert wurde, behandelt. Anschließend wurden die Haare mit Wasser gespült und getrocknet.

**Haarfärbemittel**

| | |
|---|---|
| 0,1 g | Farbstoff (A) - (F) |
| 10,0 g | Gemisch aus den Kaliumsalzen der Ölsäure und der Kokosfettsäure (im Verhältnis 1:2) |
| 10,0 g | Isopropanol |
| 10,0 g | Ammoniak (25 %ige wäßrige Lösung) |
| 69,9 g | Wasser |
| 100,0 g | |

Die so gefärbten Haarsträhnen wurden sodann bei Raumtemperatur eine Minute lang mit 1-n Salzsäure behandelt, anschließend mit einem üblichen Shampoo gewaschen und getrocknet.

Die Lab-Farbwerte der erhaltenen gefärbten Haarsträhnen wurden jeweils vor und nach der Salzsäurebehandlung mit einem Minolta-Farbmeßgerät, Typ Chromameter 100 bestimmt. Die ermittelten Lab-Farbmeßwerte sind in der nachfolgenden Tabelle 1 zusammengefaßt.

Aus der Tabelle 1 ist ersichtlich, daß die Farbmeßwerte der mit dem erfindungsgemäßen Mittel (A - D) gefärbten Haare durch Behandlung mit Säuren in wesentlich geringerem Maße verändert werden als dies bei den mit den aus dem Stand der Technik bekannten Verbindungen (E, F) gefärbten Haaren der Fall ist.

Die im Vergleich zum Stand der Technik wesentlich verbesserte Säurestabilität des erfindungsgemäßen Mittels wird noch deutlicher, wenn die Gesamtfarbänderung $\Delta Y$ nach Lin und Anderson (vgl. US-PS 5 000 755) gemäß der folgenden Formel berechnet wird:

$$Y = \sqrt{(\Delta L)^2 + (\Delta a)^2 + (\Delta b)^2}$$

Alle Prozentangaben stellen, soweit nicht anders vermerkt, Gewichtsprozente dar.

Tabelle 1

| **Farbmeßwerte der Waschversuche mit 1-n Salzsäure** | | | |
|---|---|---|---|
| Farbstoff | unbehandelt | 1-n Salzsäure | ΔY |
| 2,6-Diamino-3-(3'-azopyridyl)-pyridin (A) | L 76,5 | 77,2 | 5,1 |
| | a 4,8 | 3,4 | |
| | b 86,6 | 81,7 | |
| 2-Amino-5-[4'-N,N-bis-(β-hydroxyethyl)-amino-phenylazo]-pyridin (B) | L 78,6 | 77,3 | 3,9 |
| | a 0,6 | 3,7 | |
| | b 57,3 | 55,3 | |
| 2-Methylamino-5-[4'-N,N-bis-(β-hydroxyethyl)amino-phenylazo]-pyridin (C) | L 79,3 | 80,9 | 2,6 |
| | a 0,8 | -1,1 | |
| | b 54,3 | 53,7 | |
| 2-(β-Hydroxyethyl)-amino-5-[4'-N,N-bis-(β-hydroxyethyl)-amino-)phenylazo]-pyridin (D) | L 75,4 | 77,8 | 4,2 |
| | a 5,0 | 1,6 | |
| | b 63,8 | 63,6 | |
| 4-Amino-4'-[N,N-bis-(β-hydroxyethyl)-amino]-azobenzol (E) | L 76,3 | 71,0 | 14,5 |
| | a 5,7 | -2,0 | |
| | b 74,4 | 63,3 | |
| 3-[4'-N,N-bis-(β-hydroxyethyl)amino-phenylazo]-pyridin (F) | L 77,5 | 75,2 | 11,2 |
| | a 3,2 | 10,2 | |
| | b 69,3 | 60,8 | |

## Patentansprüche

1. Mittel zum Färben von Haaren mit einem Gehalt an für Haarfärbemittel üblichen Zusätzen, dadurch gekennzeichnet, daß es mindestens einen 3-Pyridinazofarbstoff der Formel (I) oder (II)

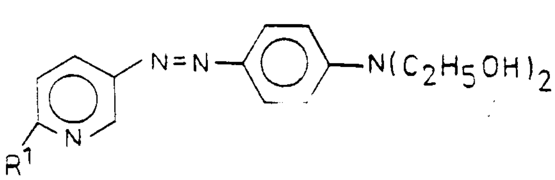

(I)

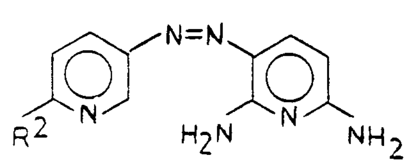

(II)

wobei $R^1$ eine Aminogruppe, eine $C_1$- bis $C_6$-Alkylaminogruppe oder eine $C_2$- bis $C_4$-Hydroxyalkylaminogruppe bedeutet und $R^2$ Wasserstoff, eine Aminogruppe, eine $C_1$- bis $C_6$-Alkylaminogruppe oder eine $C_2$- bis $C_4$-Hydroxyalkylaminogruppe ist, enthält.

2. Mittel nach Anspruch 1, dadurch gekennzeichnet, daß der 3-Pyridinazofarbstoff der Formel (I) oder (II) ausgewählt ist aus 2,6-Diamino-3-[3'-azopyridyl]-pyridin, 2-Amino-5-[4'-N,N-bis-(β-hydroxyethyl)-amino-phenylazo]-pyridin, 2-Methylamino-5-[4'-N,N-bis-(β-hydroxyethyl)amino-phenylazo]-pyridin und 2-(β-Hydroxyethyl)amino-5-[4'-N,N-bis-(β-hydroxyethyl)amino-phenylazo]-pyridin.

3. Mittel nach Anspruch 1 oder 2, dadurch gekennzeichnet, daß der 3-Pyridinazofarbstoff der Formel (I) oder (II) in einer Menge von 0,01 bis 1 Gewichtsprozent enthalten ist.

4. Mittel nach einem der Ansprüche 1 bis 3, dadurch gekennzeichnet, daß es einen pH-Wert von 3 bis 10,5 aufweist.

5. Mittel nach einem der Ansprüche 1 bis 4, dadurch gekennzeichnet, daß es in Form einer wäßrigen oder wäßrig-alkoholischen Lösung, einer Creme, eines Geles, einer Emulsion oder eines Schaumes vorliegt.

6. Mittel nach einem der Ansprüche 1 bis 5, dadurch gekennzeichnet, daß es zusätzlich einen oder mehrere direkt auf das Haar aufziehende Farbstoffe enthält.

7. Mittel nach Anspruch 6, dadurch gekennzeichnet, daß der zusätzlich enthaltene direkt auf das Haar aufziehende Farbstoff ausgewählt ist aus 2-Nitro-1,4-diaminobenzol, 2-Amino-4,6-dinitrophenol, 2-Amino-5-(β-hydroxyethyl)amino-nitrobenzol, 4-(β-Hydroxyethyl)amino-3-nitrophenol, 1-(β-Hydroxyethyl)amino-2-amino-4-nitrobenzol, 4-[(2',3'-Dihydroxypropyl)-amino]-3-nitro-trifluormethylbenzol, 1,4-Bis-[(β-hydroxyethyl)amino]-4-N-ethyl-2-nitrobenzol, 4-(β-Hydroxyethyl)amino-3-nitrotoluol, 2,5-Bis-[(β-hydroxyethyl)amino]-nitrobenzol, 2-(β-Hydroxyethyl)amino-4,6-dinitrophenol, 4-[(2',3'-Dihydroxypropyl)amino]-1-[(β-hydroxyethyl)amino]-2-nitrobenzol, 2-Amino-6-chlor-4-nitro-phenol, 4-[(β-Hydroxyethyl)amino]-2-nitro-anilin, 1-[(β-Hydroxyethyl)amino]-4-[bis-(β-hydroxyethyl)amino]-2-nitro-benzol, 4-[Bis-(β-hydroxyethyl)amino]-1-[(3'-hydroxypropyl)amino]-2-nitro benzol, 1-[(2',3'-Dihydroxypropyl)amino]-4-[N-ethyl-N-(β-hydroxyethyl)amino]-2-nitro-benzol, 4-(β-Ureidoethyl)-amino-nitrobenzol, 1-Amino-4-[(2',3'-dihydroxypropyl)-amino]-5-chlor-2-nitrobenzol, 1-Amino-2-nitro-4-[bis-(β-hydroxyethyl)amino]-benzol, Basic Violet 1 (C.I. 42 535), Basic Violet 14 (C.I. 42 510), Basic Violet 2 (C.I. 42 520), Acid Brown 4 (C.I. 14 805), Disperse Violet 4 (C.I. 61 105), 1,4,5,8-Tetraaminoanthrachinon, 1-Methylamino-4-[(β-hydroxyethyl)amino]-anthrachinon, 1,4-Diaminoanthrachinon und 1,4-[Bis-(2',3'-dihydroxypropyl)amino]-anthrachinon.

8. Mittel nach einem der Ansprüche 1 bis 7, dadurch gekennzeichnet, daß es in Form eines Haarfärbemittels mit zusätzlicher Haarfestigung vorliegt und mindestens ein in der Kosmetik üblicherweise verwendetes Polymerisat oder natürliches Polymer enthält.

9. Mittel nach Anspruch 8, dadurch gekennzeichnet, daß das Polymerisat oder das natürliche Polymer ausgewählt ist aus Polyvinylpyrrolidon, Polyvinylacetat, Polyvinylalkohol, Polyacrylsäure, Polymethacrylsäure sowie basischen Polymerisaten der Ester der Polyacrylsäure oder Polymethacrylsäure mit Aminoalkoholen, deren Salzen oder Quaternisierungsprodukten, Polyacrylnitril, Polyvinyllactamen oder Copolymerisaten aus diesen Verbindungen, Chitosan und Chitosanderivaten.

10. 3-Pyridinazofarbstoff der Formel (I) .

11. 2-Amino-5-[4'-N,N-bis-(β-hydroxyethyl)amino-phenylazo]-pyridin.

12. 2-Methylamino-5-[4'-N,N-bis-(β-hydroxyethyl)amino-phenylazo]-pyridin.

13. 2-(β-Hydroxyethyl)amino-5-[4'-N,N-bis-(β-hydroxyethyl)amino-phenylazo]-pyridin.

## Claims

1. Hair colouring agent containing additives conventional for hair colouring agents, characterized in that it contains at least one 3-pyridine azo dye of formula (I) or (II)

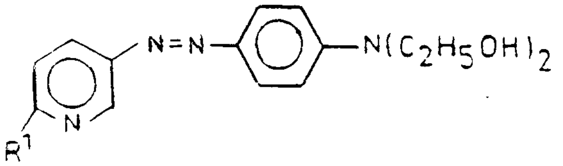

(I)

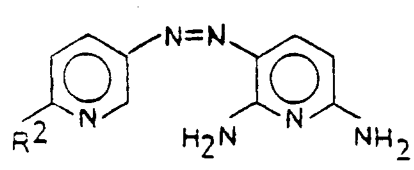

(II)

in which $R^1$ denotes an amino group, a $C_1$ to $C_6$ alkyl amino group or a $C_2$ to $C_4$ hydroxy alkyl amino group and $R^2$ is hydrogen, an amino group, a $C_1$ to $C_6$ alkyl amino group or a $C_2$ to $C_4$ hydroxy alkyl amino group.

**2.** Agent according to Claim 1, characterised in that the 3-pyridine azo dye of formula (I) or (II) is selected from 2,6-diamino-3-[3'-azopyridyl]-pyridine, 2-amino-5-[4'-N,N-bis-(β-hydroxyethyl)-amino-phenylazo]pyridine, 2-methylamino-5-[4'-N,N-bis-(β-hydroxyethyl)aminophenylazo]-pyridine and 2-(β-hydroxyethyl)amino-5-[4'-N,N-bis-(β-hydroxyethyl)amino-phenylazo]-pyridine.

**3.** Agent according to Claim 1 or 2, characterised in that the 3-pyridine azo dye of formula (I) or (II) is present in an amount of from 0.01 to 1 weight %.

**4.** Agent according to any one of Claims 1 to 3, characterised in that it has a pH value of between 3 and 10.5.

**5.** Agent according to any one of Claims 1 to 4, characterised in that it is in the form of an aqueous or aqueous-alcoholic solution, a cream, a gel, an emulsion or a foam.

**6.** Agent according to any one of Claims 1 to 5, characterised in that it additionally contains one or more direct-acting dyes.

**7.** Agent according to Claim 6, characterised in that the additionally present direct-acting dye is selected from 2-nitro-1,4-diaminobenzene, 2-amino-4,6-dinitrophenol, 2-amino-5-(β-hydroxyethyl)amino-nitrobenzene, 4-(β-hydroxyethyl)amino-3-nitrophenol, 1-(β-hydroxyethyl)-amino-2-amino-4-nitrobenzene, 4-[(2',3'-dihydroxypropyl)-amino]-3-nitrotrifluoromethylbenzene, 1,4-bis-[(β-hydroxyethyl)amino]-4-N-ethyl-2-nitrobenzene, 4-(β-hydroxyethyl)amino-3-nitrotoluene, 2,5-bis-[β-hydroxyethyl)amino]-nitrobenzene, 2-(β-hydroxyethyl)amino-4,6-dinitrophenol, 4-[(2',3'-dihydroxypropyl)amino]-1-[(β-hydroxyethyl)amino]-2-nitrobenzene, 2-amino-6-chloro-4-nitro-phenol, 4-[(β-hydroxyethyl)amino]-2-nitro-aniline, 1-[(β-hydroxyethyl)amino)-4-[bis-(β-hydroxyethyl)amino]-2-nitro-benzene, 4-[bis-(β-hydroxyethyl)amino]-1-[(3'-hydroxypropyl)amino]-2-nitro-benzene, 1-[(2',3'-dihydroxypropyl)amino]-4-[N-ethyl-N-(β-hydroxyethyl)amino]-2-nitro-benzene, 4-(β-ureidoethyl)-amino-nitrobenzene, 1-amino-4-[(2',3'-dihydroxypropyl)-amino]-5-chloro-2-nitrobenzene, 1-amino-2-nitro-4-[bis-(β-hydroxyethyl)amino]-benzene, Basic Violet 1 (C.I. 42 535), Basic Violet 14 (C.I. 42 510), Basic Violet 2 (C.I. 42 520), Acid Brown 4 (C.I. 14 805), Disperse Violet 4 (C.I. 61 105), 1,4,5,8-tetraaminoanthraquinone, 1-methylamino-4-[(β-hydroxyethyl)-amino]-anthraquinone, 1,4-diaminoanthraquinone and 1,4-[bis-(2',3'-dihydroxypropyl)amino]-anthraquinone.

**8.** Agent according to any one of Claims 1 to 7, characterised in that it is in the form of a hair colouring agent with an additional hair setting agent and contains at least one polymer conventionally used in cosmetics or a natural polymer.

**9.** Agent according to Claim 8, characterised in that the polymer or natural polymer is selected from polyvinyl pyrrolidone, polyvinyl acetate, polyvinyl alcohol, polyacrylic acid, polymethacrylic acid as well as basic polymers of the esters of polyacrylic acid or polymethacrylic acid with amino alcohols, their salts or quaternisation products, polyacrylnitrile, polyvinyl lactams or copolymers of these compounds, chitosan and chitosan derivatives.

**10.** 3-pyridine azo dye of formula (I).

**11.** 2-amino-5-[4'-N,N-bis-(β-hydroxyethyl)amino-phenylazo]-pyridine.

**12.** 2-methylamino-5-[4'-N,N-bis-(β-hydroxyethyl)amino-phenylazo]-pyridine.

**13.** 2-(β-hydroxyethyl)amino-5-[4'-N,N-bis-(β-hydroxyethyl)amino-phenylazo]-pyridine.

## Revendications

1. Agent de teinture capillaire contenant une teneur en additifs usuels pour de tels agents, caractérisé en ce qu'il contient au moins un colorant à base de 3-azopyridine de formule (I) ou (II),

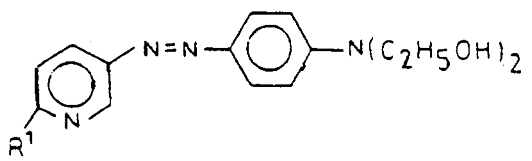

(I)

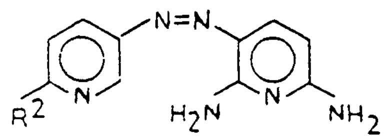

(II)

dans lesquelles $R^1$ représente un groupe amino, un groupe amino-alkyle en $C_1$ à $C_6$ ou un groupe hydroxyalkyle-amino en $C_2$ à $C_4$, et $R^2$ représente l'hydrogène, un groupe amino, un groupe amino-alkyle en $C_1$ à $C_6$ ou un groupe hydroxyalkylamino en $C_2$ à $C_4$.

2. Agent selon la revendication 1, caractérisé en ce que le colorant azoïque à base de 3-pyridine de formule (I) ou (II) est choisi parmi la 2,6-diamino-3-[3'-azopyridyl]-pyridine, la 2-amino-5-[4'-N-N-bis-(β-hydroxyéthyl)-aminophénylazo]-pyridine, la 2 méthylamino-5-[4'-N-N-bis-(β-hydroxyéthyl)aminophénylazo]-pyridine, et la 2-(β-hydroxyéthyl)amino-5-[4'-N-N-bis-(β-hydroxyéthyl)amino-phénylazo]-pyridine.

3. Agent selon la revendication 1 ou 2, caractérisé en ce que le colorant du 3-azopyridine de formule (I) ou (II) est contenu à raison de 0,01 à 1% en poids.

4. Agent selon l'une des revendications 1 à 3, caractérisé en ce qu'il présente une valeur de pH allant de 3 à 10,5.

5. Agent sel on l'une des revendications 1 à 4, caractérisé en ce qu'il se présente sous forme d'une solution aqueuse ou hydro-alcoolique, d'une crème, d'un gel, d'une émulsion ou d'une mousse.

6. Agent selon l'une des revendications 1 à 5, caractérisé en ce qu'il contient en plus un ou plusieurs colorants se fixant directement sur les cheveux.

7. Agent selon la revendication 6, caractérisé en ce que le colorant contenu en plus se fixant directement sur les cheveux est choisi parmi le 2-nitro-1,4-diaminobenzène, le 2-amino-4,6-dinitrophénol, le 2-amino-5-(β-hydroxyéthyl)amino-nitrobenzène, le 4-(β-hydroxyéthyl)amino-3-nitrophénol, le 1-(β-hydroxyéthyl)amino-2-amino-4-nitrobenzène, le 4[(2',3'-dihydroxypropyl)-amino]-3-nitro-trifluorométhylbenzène, le 1,4-bis-[β-hydroxyéthyl)amino]-4-N-éthyl-2-nitrobenzène, le 4(β-hydroxyéthyl)amino-3-nitrotoluène, le 2,5-bis-[(β-hydroxyéthyl)amino]-nitrobenzène, le 2(β-hydroxyéthyl)amino-4,6-dinitrophénol, le 4-[(2',3'-dihydroxypropyl)amino]-1-[(β-hydroxyéthyl)amino]-2-nitrobenzène, le 2-amino-6-chloro-4-nitro-phénol, le 4-[(β-hydroxyéthyl)amino]-2-nitro-aniline, le 1-[(β-hydroxyéthyl)amino]-4-(bis-(β-hydroxyéthyl)amino)-2-nitrobenzène, le 4-[bis-(β-hydroxyéthyl)amino]-1-[(3'-hydroxypropyl)amino]-2-nitro-benzène, le 1-[(2',3'-dihydroxypropyl)amino]-4-[N-éthyl-N-(β-hydroxyéthyl)amino]]-2-nitro-benzène, le 4-(β-uréidoéthyl)-amino-nitrobenzène, le 1-amino-4-[(2',3'-dihydroxypropyl)amino]-5-chloro-2-nitro-benzène, le 1-amino-2-nitro-4-[bis-(β-hydroxyéthyl)amino]-benzène, le Basic Violet 1 (C.I. 42 535), le Basic Violet 14 (C.I. 42 510), le Basic Violet 2 (C.I. 42 520), l'Acide Brown 4 (C.I. 14 805), le Disperse Violet 4 (C.I. 61 105), la 1,4,5,8-tétraaminoanthraquinone, la 1-méthylamino-4-[(β-hydroxyéthyl)amino]-anthraquinone, la 1,4-diaminoanthraquinone, et la 1,4-[bis-(2',3'-dihydroxypropyl)amino]-anthraquinone.

8. Agent selon l'une des revendications 1 à 7, caractérisé en ce qu'il se présente sous forme d'agent de teinture capillaire avec des fixateurs additionnels et contient au moins un polymère synthétique ou un polymère naturel utilisé de manière habituelle dans les cosmétiques.

9. Agent selon la revendication 8, caractérisé en ce que le polymère synthétique ou le polymère naturel est choisi parmi la polyvinylpyrrolidone, l'acétate de polyvinyle, l'alcool polyvinylique, l'acide polyacrylique, l'acide polyméthacrylique, ainsi que les polymérisats basiques de l'ester de l'acide polyacrylique ou polyméthacrylique avec des aminoalcools, leurs sels ou leurs dérivés quaternisés, le polyacrylo-nitrile, les lactames de polyvinyle, ou les copolymérisats de ces composés, du chitosane ou des dérivés du chitosane.

**10.** Colorant de 3-azopyridine de formule (I)

**11.** 2-amino-5-[4'-N,N-bis-(β-hydroxyéthyl)aminophényl-azo]-pyridine.

**12.** 2-méthylamino-5-[4'N,N-bis-(β-hydroxyéthyl)aminophényl-azo]-pyridine.

**13.** 2-(β-hydroxyéthyl)amino-5-[4'-N,N-bis-(β-hydroxyéthyl)amino-phénylazo-pyridine.